# EUROPEAN PATENT SPECIFICATION

(11) **EP 3 265 002 B1**
(45) Date of publication and mention of the grant of the patent: **20.09.2023**
(21) Application number: 16759505.7
(22) Date of filing: 03.03.2016
(51) Int. Cl.: A61B 17/28, A61B 17/29, A61B 18/14, A61B 10/00

(54) **MICROFORCEPS**
MIKROZANGE
MICRO PINCES

(30) Priority: 03.03.2015 US 201514636895
(43) Date of publication of application: 10.01.2018
(73) Proprietor: United States Endoscopy Group, Inc., Mentor, OH 44060 (US)
(72) Inventor: HAACK, Scott, Chardon, Ohio 44024 (US); ROUSH, Eric, Cleveland Heights, Ohio 44118 (US); USPENSKI, Alexis, Rock Creek, Ohio 44084 (US); WINSTANLEY, John, Madison, Ohio 44057 (US)
(74) Representative: Petraz, Gilberto Luigi
(86) International application number: PCT/US2016/020717
(87) International publication number: WO 2016/141200

(56) References cited:
- CN-A- 101 400 310
- US-A- 4 881 550
- US-A- 5 394 885
- US-A- 5 819 738
- US-A- 6 036 656
- US-A1- 2009 131 932
- US-A1- 2009 131 932
- US-A1- 2010 057 085
- US-A1- 2012 010 628
- US-A1- 2012 203 063
- US-A1- 2014 135 821
- US-A1- 2014 135 821

## Description

### Cross-Reference to Related Applications

This application claims priority to and any benefit of U.S. Patent Application No. 14/636,895, filed March 3, 2015.

### Background

The present disclosure generally relates to the field of biopsy and, more specifically, to forceps deployable through a needle.

It has been estimated that 2.8 percent of the entire adult population has pancreatic cysts (GASTROENTEROLOGY Vol. 139, No. 3, September 2010). When diagnosing such cysts to determine whether they present a risk of cancer, a doctor must examine whether the cyst is mucinous and, if so, whether it is benign or malignant. Several methods currently exist to diagnose pancreatic cysts, but all have serious shortcomings. For example, sampling of cyst fluid to test for carcinoembryonic antigen (CEA) can identify mucinous lesions with accuracy, but cannot detect malignancy because the fluid lacks cellular material. Endoscopic-ultrasound (EUS) morphology has a lower than 60 percent accuracy for detecting malignancy. Cytology, because of the small quantity of cells taken, has an even lower accuracy in determining malignancy.

While histology offers the best diagnosis, current histological methods are ineffective when sampling soft pancreatic tissue, or other more rigid tissues such as lymph node tissue, or even necrotic tissue. For example, US 2009/131932 A1 discloses a bipolar forceps can be used to seal a vessel in two locations such that the vessel can be incised at a location positioned intermediate the two seal locations. The bipolar forceps can include a cutting element which can be configured to incise the vessel. In various embodiments, the cutting element can include a sharp edge which can be moved relative to the vessel. In at least one embodiment, the cutting element can be electrically connected to a source of energy. The bipolar forceps can include first and second electrodes positioned within first and second jaw members, respectively, wherein at least one of the jaw members can include a substantially tapered profile and can be configured to pull the vessel away from the surrounding soft tissue. Such jaw members can include ridges, teeth, and/or a textured outer surface configured to grip the soft tissue and/or vessel. US 2014/135821 A1 discloses a surgical laparoscopic forceps include slidably actuatable needle lock to lock jaws of the forceps closed while holding a suture needle trapped in the jaws of the forceps. The needle lock can be a band, ring, clip or sleeve that can be selectively deployed and is sized and configured to fit in a trocar over a shaft of the forcep. US 5,819,738 A also discloses an endoscopic forceps jaw assembly for an endoscopic instrument includes a pair of articulating opposed jaws each having a jaw cup. The jaw cups are provided with teeth which increase in height from the proximal end of the jaw cup to the distal end. The jaws are rotatable relative to each other about a common axis and define an open position and a closed position. When the jaws are rotated toward the closed position, the teeth interleave substantially simultaneously. An endoscopic instrument utilizing the jaws of the invention is also provided and further includes an actuation handle, a tubular member, and a control member. The jaw assembly is coupled to the distal end of the control member and rotatably coupled to the distal end of the tubular member, such that the pair of articulable opposed jaws open and close by rotating in response to movement of the control member relative to the tubular member. The jaws may also be electrically conductive and coupled to an electrocautery voltage supply. Additionally, CN 101400310 A discloses a treatment endoscope including a sheath having a flexibility; one or plural arm members having a bending part that projects out from a front end of the sheath and performs bending actions; an open/close mechanism which directs the arm member from a direction along a central axis of the sheath to a direction deviated from the central axis of the sheath, and from a direction deviated from the central axis of the sheath to a direction along the central axis of the sheath; and a viewing device and an illuminating member that are disposed to the front end side of the sheath. The treatment endoscope can make the front end of the arm member have relative displacement with respect to said viewing device and said illuminating member via at least one of the bending action of the bending part and direction deviation of the arm member by said open/close mechanism. US 2012/010628 A1 discloses sheaths for medical instruments cover wrist mechanisms to provide a barrier to infiltration of biological material into the instrument, electrical isolation of energized portions of the instrument, seal the instrument to help maintain cavity pressure within a patient, or reduce the chance that two jointed instruments will tangle during a medical procedure. Traditional forceps, deployed via endoscopic or laparoscopic means are either not strong enough to pierce the outer tissue wall or are too large to effectively pierce the tissue wall without causing unwarranted damage to the tissue. On the other hand, needle biopsies, which may safely pierce the outer wall, do not obtain a sufficient tissue sample and are difficult to precisely control. It is thus difficult to obtain samples from the periphery of a lesion, where abnormal cells most often appear. A needle must often be inserted and retracted several times to obtain a sufficient sample for histology. For example, current practice may require five needle passes for pancreatic masses and 3 for lymph nodes.

### Summary

There is thus a need in the art for a device that can easily and efficiently pierce a harder tissue wall and obtain a sufficient tissue sample within. The invention is defined in claim 1. Further embodiments of the invention are defined in the dependent claims. Methods are not claimed. The present application relates to a forceps device. According to one aspect of the disclosure, a forceps device includes a first jaw, a second jaw rotationally connected to the first jaw by a clevis pin of a clevis, a first linking piece rotationally connected to the first jaw, a second linking piece rotationally connected to the second jaw, and a box slider rotationally connected to both the first and second linking pieces. Moving the box slider distally causes the first and second jaws to rotate to an open position. Moving the box slider proximally causes the first and second jaws to rotate to a closed position. The jaws are deployable from within a needle.

According to another aspect of the disclosure, a forceps device includes a first fixed jaw and a second jaw movable relative to the first jaw, wherein the jaws are deployable from within a needle.

According to a further aspect of the disclosure, a forceps device includes jaws with teeth that increase in height from the proximal end of the jaws to the distal end of the jaws.

According to yet another aspect of the disclosure, a histology and cytology device includes a forceps apparatus deployable from within and removable from the bore of a needle and a suction device. Suction may be achieved through the bore of the needle when the forceps apparatus is removed.

### Brief Description of Drawings

Figures 1a and 1b are side views of an exemplary microforceps apparatus in closed and open positions respectively.
Figure 2 is an isometric view of an exemplary forceps assembly.
Figure 3 is an exploded isometric view of the exemplary forceps assembly of Figure 2.
Figures 4a and 4b are cutaway side views of another exemplary forceps assembly in closed and open positions, respectively.
Figure 5 is an exploded isometric view of the exemplary forceps assembly of Figures 4a and 4b.
Figures 6-9 are isometric views of various exemplary forceps assemblies.
Figure 10 is an isometric view of an exemplary fenestration of an exemplary forceps assembly.
Figures 11a and 11b are side views of an exemplary bottom jaw configuration with and without exemplary measurements, respectively.
Figures 12a and 12b are side views of an exemplary top jaw configuration without and with exemplary measurements, respectively.
Figures 13a-13d are side views of the multiple layers of an exemplary bottom jaw.
Figure 14 is an isometric view of an exemplary bottom jaw having multiple layers.
Figures 15a-15f are side views of the multiple layers of an exemplary top jaw.
Figure 16 is an isometric view of an exemplary top jaw having multiple layers.
Figure 17 is a cross-sectional view of an exemplary lesion with an exemplary needle and microforceps apparatus deployed within.

### Detailed Description

The present disclosure describes embodiments of a microforceps apparatus, which includes a forceps at one end. The microforceps apparatus is small enough to be deployed through a needle, which allows for efficient deployment into tissue with a harder outer wall, such as tissue of pancreas or lymph nodes. In some embodiments, both jaws of the forceps move relative to each other. In some embodiments one jaw as fixed and the other is movable. In some embodiments, the microforceps device includes progressively-sized teeth for increased grasping and tearing strength. The proximal-most teeth slope away from each other to create a gap that prevents the proximal teeth from meshing and lessens wear on the teeth. In further embodiments, after a histological sample is taken using the forceps, the forceps may be removed out from the needle and a cytological sample may be taken using suction through the needle.

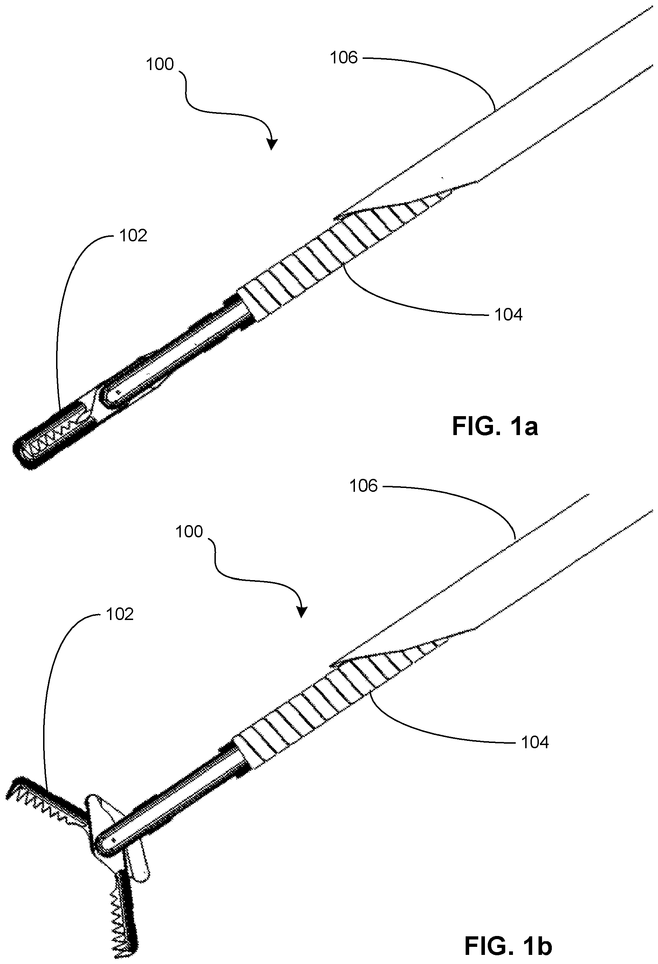

Figures 1a and 1b illustrate an exemplary embodiment of a microforceps apparatus 100 in deployed closed and open states, respectively. The microforceps apparatus 100 includes a forceps assembly 102 for grasping, tearing and/or holding tissue. The proximal end of the forceps assembly 102 is attached to the distal end of a spring sheath 104. In its deployed state (as shown in the Figures 1a and 1b), the proximal end of the spring sheath 104 is disposed within a hollow needle 106. The spring sheath 104 and forceps assembly 102 may be fully retracted into and deployed from the needle 106 using a handle (not pictured) or other suitable control mechanism at or near the proximal end of the microforceps apparatus 100.

The microforceps apparatus 100 may be deployable through a lumen, cannula, catheter, endoscope, laparoscope or the like. The needle 106 of the microforceps apparatus 100 allows a user to deploy the apparatus into tissue (e.g., the pancreas or lymph nodes) multiple times using the forceps assembly 102 within to obtain multiple tissue samples before removing the needle 106. The needle 106 may be the type of hollow needle used for fine needle aspiration (FNA) cytology. FNA typically results in a core sample of tissue in an attempt to ensure that a "false positive" is avoided where healthy tissue is only sampled instead of potentially malignant tissue. This is achieved by preventing healthy cells from entering the stylet of the needle once it is deployed. The diameter of the spring sheath 104 and forceps assembly 102 (in a closed state) should not exceed the inner diameter of the needle 106 so that the spring sheath 104 and forceps assembly 102 may deploy from and retract into the needle 106. Typically, FNA needles have a 19 gauge (approximately 0.9 mm) diameter channel. Thus, if the typical FNA needle size is used, the spring sheath 104 and forceps assembly 102 should have a diameter no larger than 0.84 mm. These sizes are exemplary and not meant to limit the scope of the present disclosure as it is contemplated that larger gauge needles (and thus larger diameter needles) and/or smaller gauge needles (with a smaller forceps assembly) may be employed in the present invention.

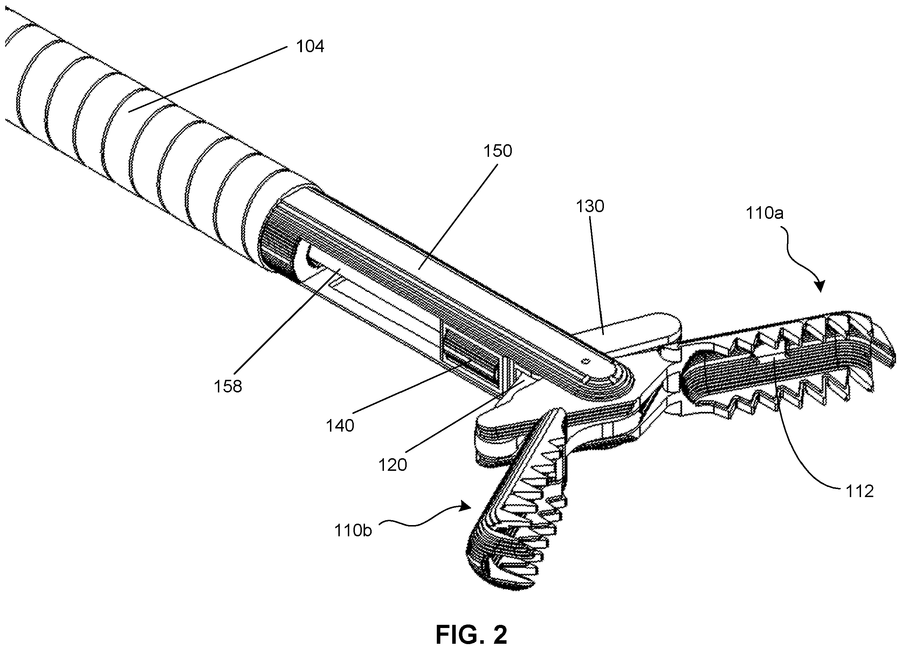

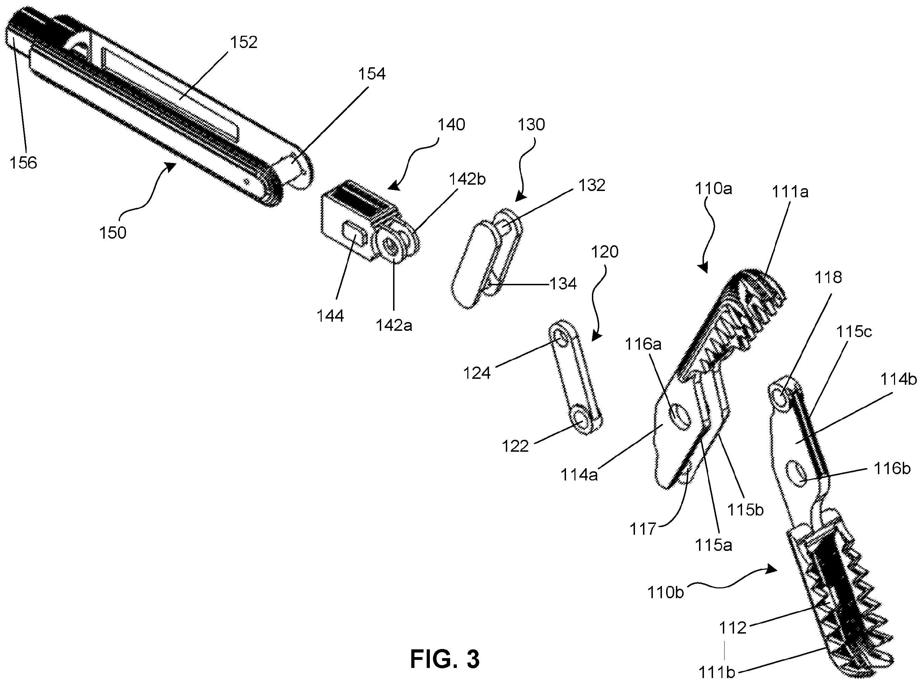

Figures 2 and 3 depict the exemplary forceps assembly 102 in greater detail. The forceps assembly 102 includes a pair of jaws 110a and 110b. Each of the jaws 110a and 110b includes a distal mandible part 111a and 111b, respectively, and a proximal arm part 114a and 114b, respectively. The mandibles 111a and 111b are shaped as opposing cups, such that when the jaws 110a and 110b are in a closed position, there is a volume between the mandibles 111a and 111b. In some embodiments, one or both of the mandibles 111a and 111b includes a fenestration, such as fenestration 112, in the cup of the mandible. The fenestration 112 allows air to escape from inside the mandibles 111a and 111b so that a buildup of air within the mandibles 111a and 111b does not inhibit tissue acquisition.

The arm 114a of jaw 110a includes two spaced walls 115a and 115b, with the space between the walls 115a and 115b being wide enough to accept a single wall 115c of arm 114b of jaw 110b between. Each wall 115a, 115b and 115c of arms 114a and 114b includes a hole, for example holes 116a and 116b. The arm 114a further includes cylindrical pillar 117 spanning between the inner sides of the walls 115a and 115b and connects to 122 on the single link 120. The arm 114b further includes an additional linking hole 118 at its proximal end that connects to 132 on linkage 130.

A first linking piece 120 includes a first linking hole 122. The first linking piece is thin enough to fit between the walls of the arm 114a. When assembled, the first linking piece 120 is rotationally connected by a cylindrical pillar 117 spanning between the inner sides of the walls 115a and 115b inserted through the first linking hole 122. The first linking piece also includes a second linking hole 124 for further connection to linking pin 134 on linkage 130.

A second linking piece 130 includes a first linking pin 132 that fits through the linking hole 118 of the arm 114b to rotationally connect the second linking piece 130 to the arm 114b. The second linking piece 130 also includes a second linking pin 134 for further connection to both linkage hole 124 and linking members 142a and 142b.

A box slider 140 includes pair of linking members 142a and 142b, each having a hole. The box slider also includes one or more guide members, such as guide member 144, on one or more sides of the box slider 140. The box slider 140 is placed within a clevis 150 having one or more guide channels, for example guide channel 152, such that a respective guide member 144 fits within the guide channel 152. The box slider 140 may then slide from the distal end of the guide channel 152 to the proximal end of the guide channel 152 (or vice versa).

A clevis pin 154 fits between the holes 116a and 116b of the jaws 110a and 110b to rotationally secure the jaws 110a and 110b to the clevis 150. The second linking hole 124 of the linking piece 120 is inserted between and aligned with the holes of the linking members 142a and 142b of the box slider 140. The second linking pin 132b of the second linking piece 130 fits through all three holes 116a, 116b, 124, thus rotationally securing the box slider 140 to both linking pieces 120 and 130. In this arrangement, when the box slider 140 slides toward the distal end of the clevis 150, the linking pieces 120 and 130 are pushed apart, in turn causing the jaws 110a and 110b to rotate apart from each other into an open position. Conversely, when the box slider 140 slides toward the proximal end of the clevis 150, the linking pieces 120 and 130 are drawn together, causing the jaws 110a and 110b to rotate toward each other into a closed position.

The clevis 150 is attached the distal end of the spring sheath 104, which fits around a collar 156 at the proximal end of the clevis 150. A drive wire 158 controls forward and backward movement of the box slider 140 within the clevis 150. The drive wire 158 should be small enough to move through the diameter of the spring sheath 104. In one exemplary embodiment the drive wire is a 0.0115" OD wire. In one embodiment the drive wire is made from nickel titanium (i.e., Nitinol). The drive wire 158 may be attached to the box slider 140 via weld, and the box slider 140 may include a hole for receiving the distal end of the drive wire 158, in which the drive wire 158 is welded or otherwise affixed to the box slider 140.

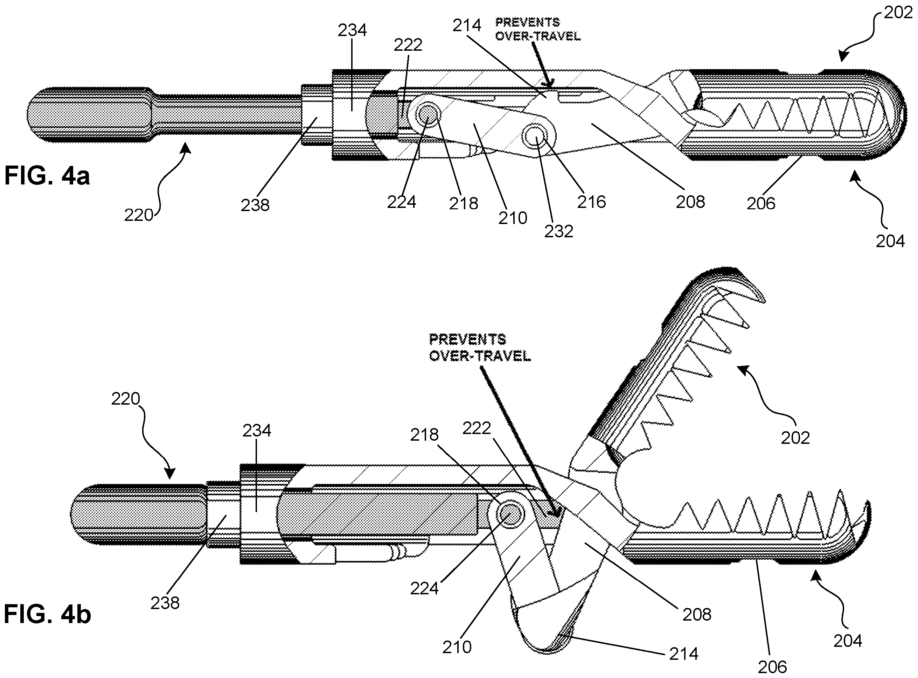

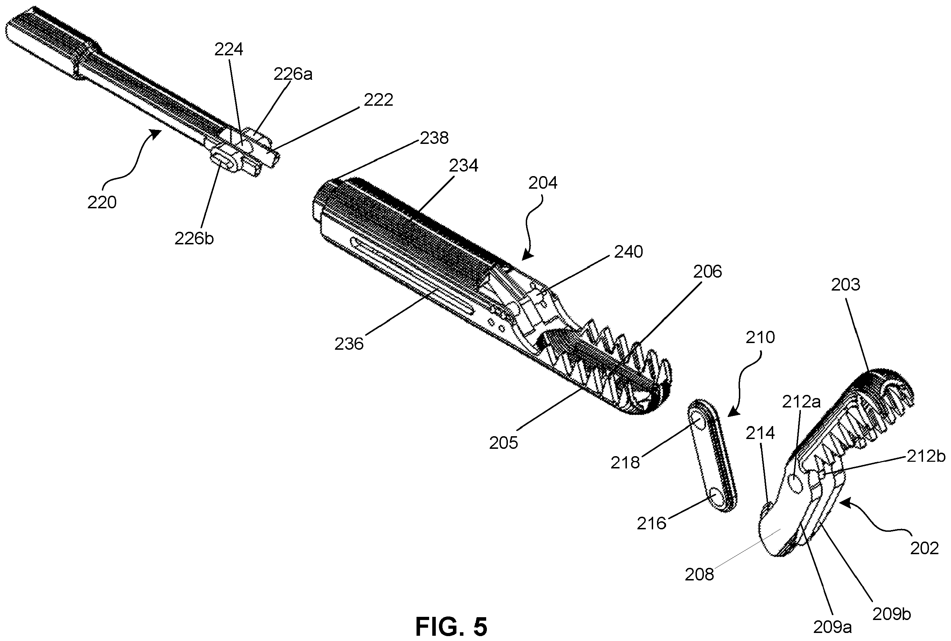

Figures 4-5 show another exemplary forceps assembly. Unlike the previously described embodiment, the forceps assembly of Figures 4-5 has one movable jaw 202 and one fixed jaw 204. Similar to the previous embodiment, the jaws 202 and 204 include distal mandible parts 203 and 205, respectively, which are shaped as opposing cups. When the mandibles 203 and 205 are in a closed position, they create a volume between them. In some embodiments, one or both of the mandibles 203 and 205 includes a fenestration or weep hole, such as fenestration 206 in the cup of the mandible. In the embodiment of Figures 4-5, the movable jaw 202 includes an proximal arm 208, which has two spaced walls 209a and 209b, with the space between the walls 209a and 209b being wide enough to accept a linking piece 210. Each wall 209a and 209b of the arm 208 includes a hole, 212a and 212b, respectively. The arm 208 further includes a cylindrical pillar 232 spanning between the inner sides of the walls 209a and 209b. Each wall 209a and 209b of the arm 208 also includes a stop projection, for example stop projection 214, at the proximal end of the wall.

The linking piece 210 includes a first linking hole 216. The linking piece 210 is thin enough to fit between the walls of the arm 208. When assembled, the linking piece 210 is rotationally connected via cylindrical pillar 232 and through the first linking hole 216. The linking piece 210 also includes a second linking hole 218 for further connection to clevis pin 224.

A drive arm 220 includes a clevis 222 with a clevis pin 224 at its distal end. The drive arm 220 also includes one or more guide members, such as guide members 226a and 226b, on one or more sides of the drive arm 220.

The fixed jaw 204 includes a clevis pin 240 proximal to the cup of the jaw. The clevis pin 240 rotationally secures the movable jaw 202 to the fixed jaw 204 by placing the pin 240 through the holes 212a and 212b of the movable jaw 202. The drive arm 220 is inserted within an enclosure 234 at the proximal end of the fixed jaw 204. The enclosure 234 includes one or more guide channels, for example guide channel 236, such that a respective guide member 226a, 226b fits within the guide channel 236. The drive arm 220 may then slide from the distal end of the guide channel 236 to the proximal end of the guide channel 236 (or vice versa).

The clevis pin 224 of the drive arm 220 is inserted into the second linking hole 218 of the linking piece 210, thereby rotationally securing the drive arm 220 to the linking piece 210. In this arrangement, when the drive arm 220 slides toward the distal end of the enclosure 234, the linking piece 210 and proximal end of the movable jaw 202 are pushed together and out away from the enclosure 234, in turn causing the distal end of the movable jaw 202 to rotate apart from the distal end of the fixed jaw 204. Conversely, when the drive arm 220 slides toward the proximal end of the enclosure 234, the linking piece 210 and proximal end of the movable jaw 202 are pulled apart and into the enclosure 234, causing the movable jaw 202 to rotate toward the fixed jaw 204 and into a closed position.

The fixed-arm forceps assembly of Figures 4-5 includes mechanisms to prevent over-travel of the movable jaw 202 when it is opened and closed. Over-travel (i.e., moving past its intended final position) may cause the jaw 202 to become stuck in an open or closed position. If stuck in closed position, the jaws 202 and 204 can no longer grab tissue and may need to be fully retracted and repaired or replaced, which can greatly increase procedure time. If stuck in an open position, it may become impossible to retract the forceps, which can lead to seriously complications that may require surgery to correct.

In order to prevent over-travel when closing the jaws 202 and 204, the movable jaw 202 includes stop projections, such as stop projection 214. Over-travel is prevented when closing the jaws 202 and 204 as follows. As the movable jaw 202 is rotated into a closed position and the stop projection 214 bumps up against the inside of the enclosure 234, which prevents the jaw 202 from rotating further.

To prevent over-travel when opening the jaw 202, the distal ends of the clevis 222 move toward the back of the arm 208 of the movable jaw 202 as the drive arm 220 is pushed forward. Once the drive arm 220 is pushed far enough, it will bottom against the arm 208 and prevent further forward motion of the drive arm 220.

As another measure for preventing over-travel when opening the jaw 202, the proximal end of the fixed jaw 204 includes a collar 238. The proximal end of the drive arm 222 has a larger circumference than its distal end (where the clevis 222 is located). The circumference of the proximal end of the drive arm 220 is larger than the circumference of the collar 238, which prevents the drive arm from moving further into the enclosure 234, thereby the jaw 202 is blocked from opening further and into an over-travel position.

The collar 238 also has a circumference designed to fit within a spring sheath (not shown) as described for earlier embodiments. The spring sheath may be attached to the collar and/or proximal end of the fixed jaw 204 by a weld or appropriate attachment mechanism. Movement of the drive arm 220 back and forth in the enclosure 234 may be effectuated by a drive wire (not shown) as described above for previous embodiments. The drive wire may be attached to the drive arm 220 via weld (or any other suitable connection as is known), and the drive arm 220 may include a hole for receiving the distal end of the drive wire, in which the drive wire is welded or otherwise affixed to the drive arm 220.

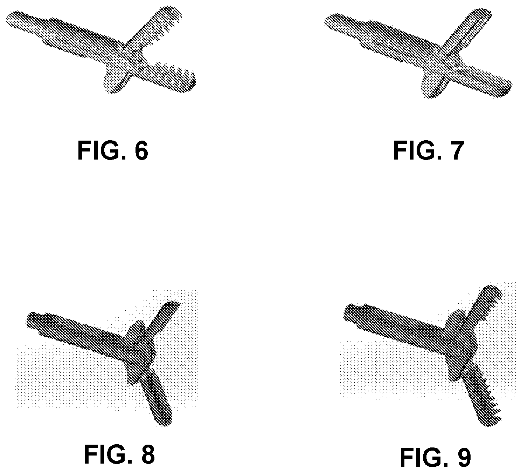

The above described embodiments pertain to drive mechanisms and configurations for opening and closing the forceps without regard to the type of jaws on the forceps. Figures 6-9 generally depict the above-described configurations having smooth jaws (Figures 7 and 8), and serrated jaws with teeth (Figures 6 and 9) and the above described drive mechanisms will operate with the smooth jaw configurations described below.

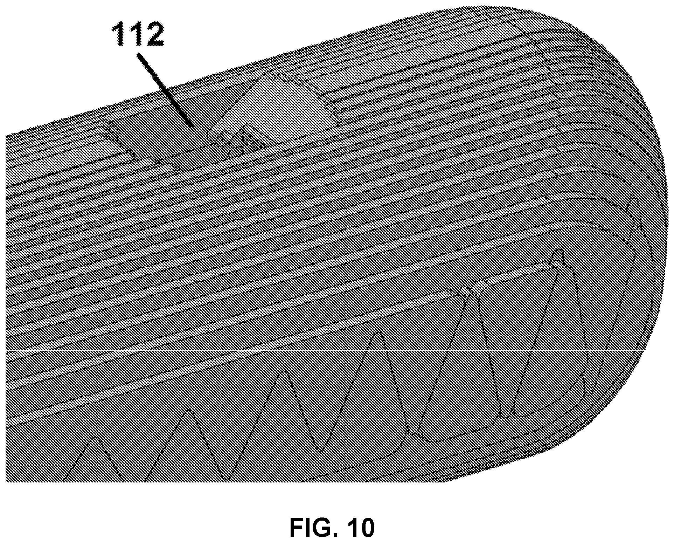

Figure 10 depicts one detailed embodiment of the previously discussed fenestration 112. In the depicted embodiment, the distal end of the fenestration 112 is angled inward (toward the inside of the cup) to prevent an edge or point (e.g., on the needle 106 or in tissue) from catching on a edge of the fenestration 112. The angle helps to deflect any contact the fenestration 112 may encounter thus enabling the jaw assembly to function as intended. Either of the top and/or bottom jaws may include such an angled edge, and the angle of the edge may be the same or different for each fenestration. An angled fenestration may be used in conjunction with any of the other jaw embodiments described herein, for example, fenestration 206 of the fixed jaw 204.

Because the exemplary forceps described above are small enough to fit through an FNA needle, the forceps may lack sufficient strength to grasp, tear and retain a tissue sample. Thus, as described below, it may be beneficial to include teeth on the jaws specifically arranged to maximize the grip of the forceps.

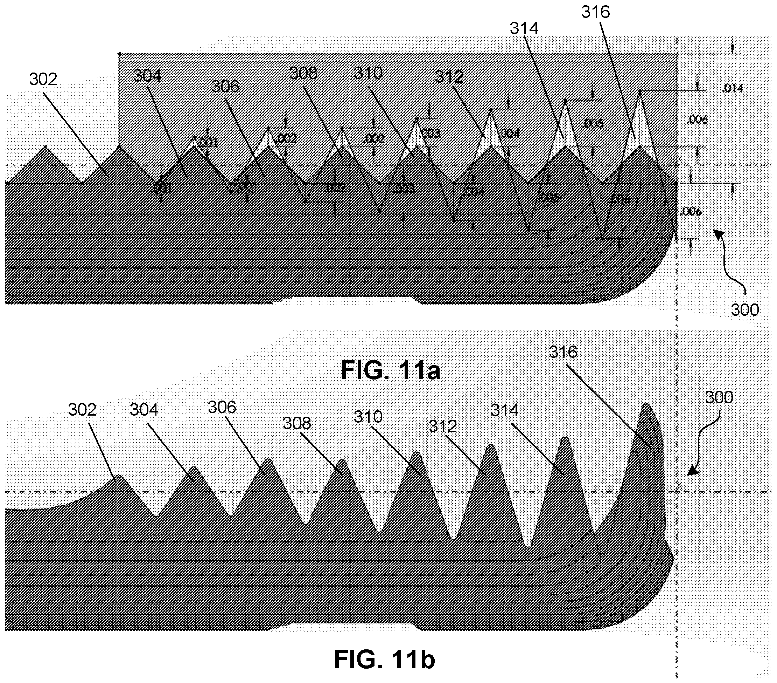

Figures 11-12 depict an exemplary tooth design with teeth that progressively increase in size from the proximal end of the jaw to the distal end of the jaw. For the exemplary bottom jaw 300 of Figures 11a and 11b, a first proximal tooth 302 protrudes from the base of the jaw. In some embodiments the proximal side of the first proximal tooth 302 slopes downward toward the proximal end of the jaw 300. This downward slope creates a gap at the back of the jaw 300 (in other words, the proximal-most tooth 302 never substantially meshes with its counterpart on an opposing jaw). Additionally, this gap prevents the proximal teeth (e.g. tooth 302) from meshing when the jaws are closed, which could cause wear on the jaws and even cause the jaws to become stuck in a closed state.

As seen in Figures 11-12, in this exemplary embodiment, a second-most proximal tooth 304 is .001 in longer than the proximal-most tooth 302. The valley between the teeth 302 and 304 is sized to receive a tooth of similar size to tooth 304. A third tooth 306 is another .001 in longer, being .002 in longer than the proximal-most tooth 302. The valley between the teeth 304 and 306 is also sized to receive a tooth of similar size to tooth 304. A fourth tooth 308 is not longer, but the same size as tooth 306. The valley between teeth 306 and 308 is sized to receive a tooth of similar size to tooth 306. Then, moving toward the distal end of the jaw 300, each tooth is .001 in longer than the previous tooth. Thus, tooth 310 is .003 in longer than the proximal-most tooth 302, tooth 312 is .004 in longer than tooth 302, tooth 314 is .005 in longer, and distal-most tooth 316 is .006 in longer. The valleys between each of those teeth similarly increase in length by .001 in moving toward the distal end of the jaw. Finally, the distal side of the distal-most tooth 316 includes a .006 in half-valley for receiving a distal-most tooth of the opposing jaw.

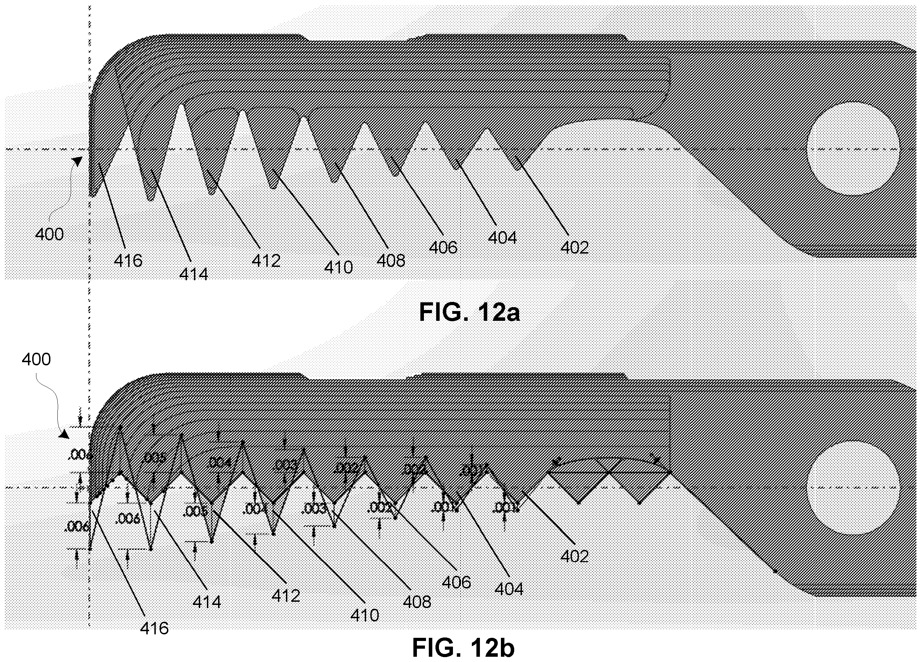

For the exemplary top jaw 400 of Figures 12a and 12b, a first proximal tooth 402 protrudes from the base of the jaw. As described above for the bottom jaw 300, the proximal side of the first proximal tooth 402 slopes downward toward the proximal end of the jaw 400. This slope creates a gap at the back of the jaw 400 (in other words, the proximal-most tooth 402 never substantially meshes with its counterpart on an opposing jaw). This gap prevents the proximal teeth (e.g. tooth 402) from meshing when the jaws are closed, which could cause wear on the jaws and even cause the jaws to become stuck in a closed state.

1 inch = 25.4 mm. In this embodiment, the proximal-most tooth 402 is .001 in larger than its opposing counterpart tooth 302 (i.e., there is no tooth similarly sized to tooth 302 on the top jaw). A second-most proximal tooth 404 is the same size as tooth 402. The valley between the teeth 402 and 404 is sized to receive opposing tooth 304. A third tooth 406 is .001 in longer, being .002 in longer than the shortest tooth 302 of the opposing jaw 300. The valley between the teeth 404 and 406 is sized to receive tooth opposing 306. Then, moving toward the distal end of the jaw 400, each tooth is .001 in longer than the last. Thus, tooth 408 is .003 in longer than the shortest tooth 302, tooth 410 is .004 in longer than tooth 302, tooth 412 is .005 in longer, and tooth 414 is .006 in longer. The distal most tooth 416 is the same length as tooth 414, also being .006 in longer than the shortest tooth 302.

In the above described exemplary embodiment, the teeth increase linearly in height from a proximal point to the distal ends of the jaw. In some embodiments the proximal point may be the most proximal tooth (i.e., all teeth increase in height with respect to the previous tooth). In some embodiments several proximal teeth have the same height and only a few of the distal teeth increase in height with respect to one another. In further embodiments only the distal-most tooth is longer than the more proximal teeth. In even further embodiments the increase in height from tooth-to-tooth may be non-linear, for example parabolic or arbitrary. In each of these embodiments, the opposing jaw may include valleys between each tooth to receive a corresponding tooth from an opposing jaw. It is not necessary the all teeth mesh at substantially the same time when the jaws are closed. In fact, the proximal-most tooth of each jaw may not mesh at all with opposing jaw, so as to prevent grinding and wear as the jaws are opened and closed.

In further embodiments described below, the teeth of the top and bottom jaws may include one or more sub-teeth. The inside of the cups of jaws further include a stacked layer pattern that has an increased surface area that helps hold a tissue sample inside the cups. The layer variations further act as smaller teeth to help grip and maintain a sample in place.

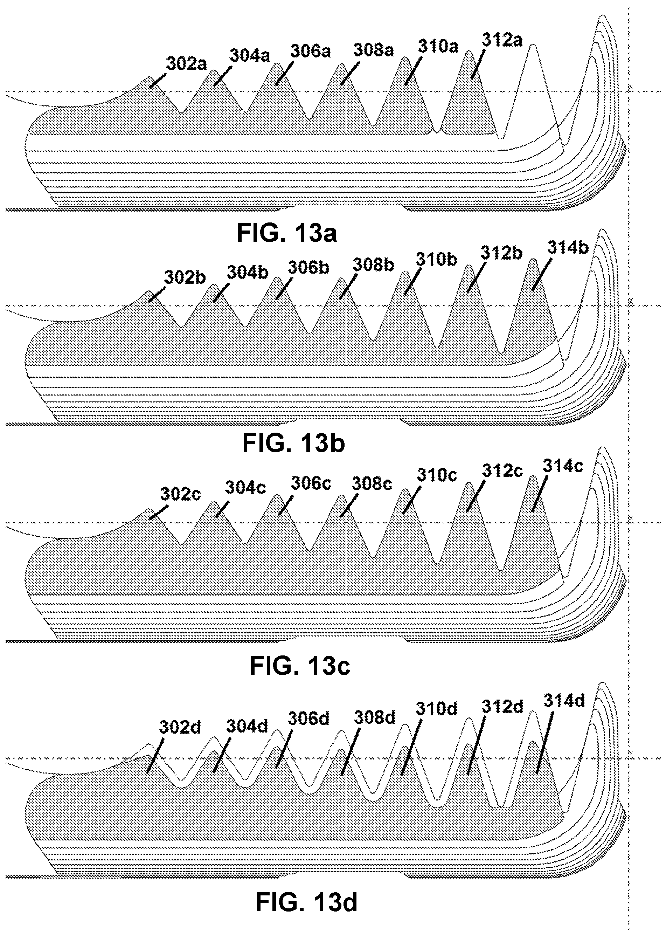

In the embodiment of Figures 13a-13d, teeth 302, 304, 306, 308, 310, and 312 are comprised of four sub-teeth designated by layers a, b, c and d (tooth 314 only has three sub-teeth b, c and d). The outermost sub-tooth layer is denoted as layer "a" while the innermost sub-tooth layer is denoted as layer "d." Sub-tooth layer "d" forms the inner wall of the cup of the jaw 300. In some embodiments each sub-tooth may have its own serration pattern by varying layers a-d (e.g., in the exemplary jaw 300, tooth 314 does not have a layer "a" and instead starts on layer "b").

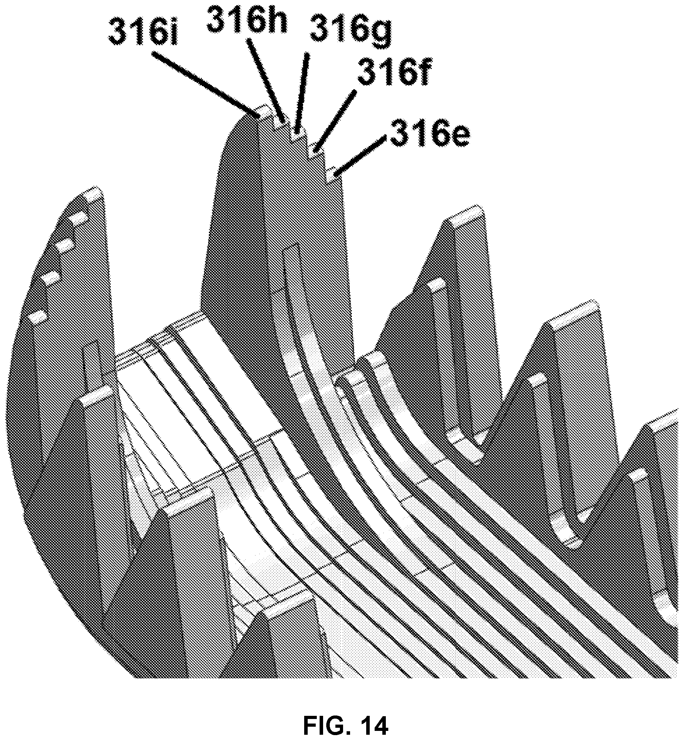

Turning to Figure 14, the front tooth 316 of the bottom jaw 300 includes sub-tooth layers 316e-316i that form an arc of serrated teeth. Because the design of the exemplary jaw 300 is symmetrical, the sub-tooth and tooth formations will be identical on the opposite side of the same jaw 300. In other embodiments, the bottom jaw 300 may have a functional asymmetrical layout and formation using the same manufacturing method.

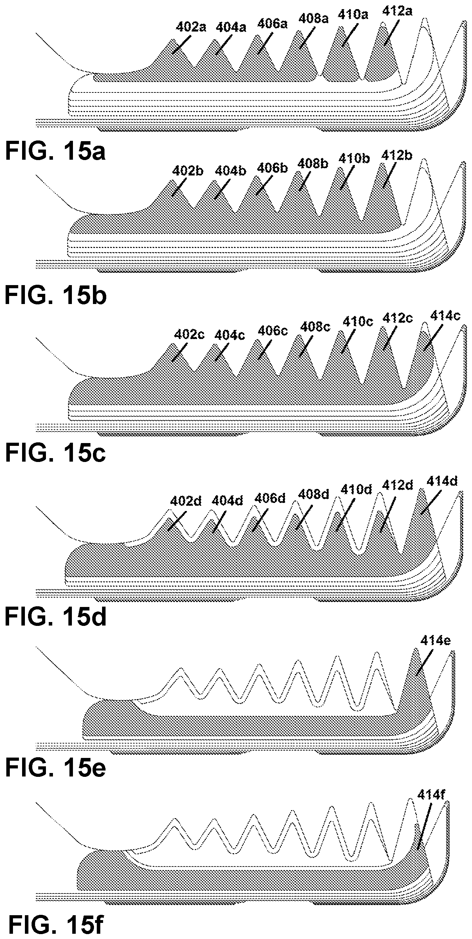

For the exemplary top jaw 400 of Figures 15a-15f (shown facing upward) each designated tooth is again includes one or more sub-teeth. In this embodiment, teeth 402, 404, 406, 408, 410, 412 and 414 are comprised of four or more sub-teeth designated by layers "a" through "n". The outermost sub-tooth layer is layer "a" while the innermost sub-tooth layer is "d" (for teeth 402, 404, 406, 408, 410, and 412), and "e" (for tooth 414). Sub-tooth layers "d" and "e" form the inner wall of the cup of the jaw 400. In some embodiments each sub-tooth may have its own serration pattern by varying layers a-n (e.g., in the exemplary jaw 400, tooth 414 does not have a layer "a or b" and starts on layer "c").

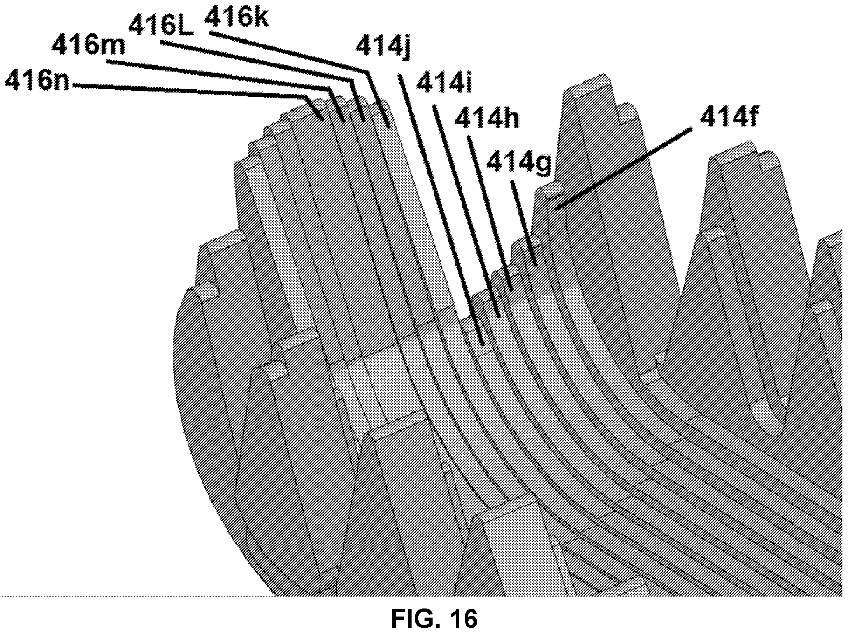

Turning to Figure 16, layers 414f-414j are supporting serrations for tooth 414 that also help form the front lip on the distal end of the jaw 400. Tooth 416 is a single tooth at the front of the jaw 400, its sub-tooth layers forming an arc of serrated teeth. Because the design of the exemplary jaw 400 is symmetrical, the sub-tooth and tooth formations will be identical on the opposite side of the same jaw 400. In other embodiments, the top jaw 400 may have a functional asymmetrical layout and formation using the same manufacturing method.

The forceps assemblies of the above embodiments may be made from any suitable material, such as nickel, Valloy-120^{™} (a nickel-cobalt alloy), or a polymeric material. In one embodiment, the components of the forceps assembly are separately manufactured and assembled. In another embodiment, the entire forceps assembly is "grown" via a method called Physical Vapor Deposition (PVD) making the individual components interlocked with one another. Such a procedure is described in U.S. Patent No. 7,291,254.

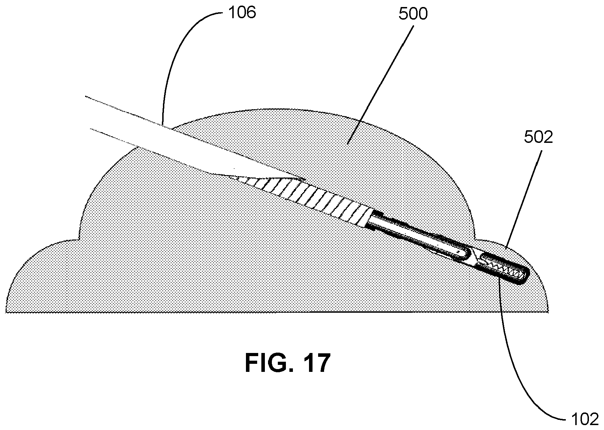

Turning to Figure 17, a cross section of an exemplary lesion 500. The lesion 500 may be any lesion of any organ of a human or other animal body. The lesion 500 includes a peripheral aspect 502. An exemplary microforceps apparatus having a forceps assembly 102 and needle 106 is shown inserted into the lesion 500 and deployed to reach the peripheral aspect 502. When the needle 106 is inserted into the lesion 500 a pre-loaded stylet provided with the needle will be in place. Once the desired location has been reached, the sytlet will be removed and the microforceps inserted through the lumen of the needle. The forceps assembly 102 will be in an undeployed state. It is exemplary that the stylet may be removed from the needle prior to insertion and replaced with the forceps assembly 102.

In some embodiments, the forceps assembly 102 itself may serve a functional stylet to perform the above described procedure. As such, the forceps assembly 102 may create a capillary pull or vacuum to create a better aspirate for cytology. The forceps assembly 102 has the ability to open slightly and create static pressure against the walls of the lumen of needle 106. Thus, the forceps assembly 102 may create a better pull or vacuum because the lumen may be better occluded with this type of action.

Once the needle 106 penetrates the lesion 500, samples may be gathered from around the peripheral aspect 502, which is typically where abnormal cells reside. In some embodiments, histological sampling via the forceps assembly 102 may be followed by or used intermittently with or used in conjunction with cytology sampling via suction. For example, the forceps assembly 102 may be fully retracted into the needle and out through the proximal end of the lumen, catheter, or cannula, through which the microforceps device is deployed. A suction device (not shown) may then be connected to the proximal end of the needle, lumen, catheter, or cannula, to suck cells inside. The ability to reach peripheral cells and well as the histological/cytological combination may increase cellular yield and lead to more accurate test results.

While the present invention has been illustrated by the description of embodiments thereof, and while the embodiments have been described in considerable detail, it is not the intention of the applicants to restrict or in any way limit the scope of the invention to such details. Additional advantages and modifications will readily appear to those skilled in the art. Accordingly, departures may be made from such details without departing from the scope of the applicant's exemplary embodiments of the invention.

## Claims

1. A forceps device (100) comprising:
a needle (106); and
a forceps assembly (102) having
a first jaw (110a, 300) comprising two spaced walls (115a, 115b) and a cylindrical pillar (117) spanning between the inner sides of said walls (115a),
a second jaw (110b, 400) opposed to and rotationally connected to the first jaw by a clevis (150),
a first linking piece (120) rotationally connected to the first jaw,
a second linking piece (130) rotationally connected to the second jaw, and
a box slider (140) rotationally connected to both the first and second linking pieces and comprising a pair of linking members (142a, 142b), each having a hole;
wherein the first linking piece (120) comprises a first linking hole (122) and a second linking hole (124), wherein the second linking piece (130) comprises linking pins (132,134); wherein said linking pins (132,134) are configured for the connection to the second linkage hole (124) and to the linking members (142a, 142b),
wherein the first linking hole (122) is rotationally connected to the cylindrical pillar (117) of the first jaw,
wherein the box slider (140) comprises at least one guide member (144) sized to fit and slide horizontally within a guide channel (152) of a clevis (150) in which the box slider is placed.
wherein moving the box slider (140) distally causes the first and second jaws to rotate to an open position, moving the box slider (140) proximally causes the first and second jaws to rotate to a closed position, and the jaws are configured to be deployed from within the needle (106); and
wherein the forceps device (100) is configured to be deployed from within a lumen, cannula, catheter, endoscope, or laparoscope.

2. The forceps device of claim 1, the jaws comprising smooth cups.

3. The forceps device of claim 1, each jaw comprising a least one tooth (302, 402).

4. The forceps device of claim 3, wherein the at least one tooth of each jaw includes a plurality of teeth that increase in length from a proximal end of the jaw (300, 400) to a distal end of the jaw (300, 400).

5. The forceps device of claim 4,
the first jaw (300) having
a first tooth (302);
a second tooth (304) distal to and longer than the first tooth,
a third tooth (306) distal to and longer than the second tooth,
a fourth tooth (308) distal to and the same length as the third tooth,
a fifth tooth (310) distal to and longer than the fourth tooth,
a sixth tooth (312) distal to and longer than the fifth tooth,
a seventh tooth (314) distal to and longer than the sixth tooth, and
an eight tooth (316) distal to and longer than the seventh tooth; and
the second jaw (400) having
a first tooth (402) longer than first tooth of the first jaw;
a second tooth (404) distal to and the same length as the first tooth,
a third tooth (406) distal to and longer than the second tooth,
a fourth tooth (408) distal to and longer than the third tooth,
a fifth tooth (410) distal to and longer than the fourth tooth,
a sixth tooth (412) distal to and longer than the fifth tooth,
a seventh tooth (414) distal to and longer than the sixth tooth, and
an eight tooth (416) distal to and the same length as the seventh tooth.

6. The forceps device of claim 1, at least one of the first and second jaws having a fenestration (112).

7. The forceps device of claim 6, wherein a distal end of the fenestration (112) is angled with respect to an outer surface of the jaw having the fenestration (112).

8. The forceps device of claim 1, the box slider (140) being connected to a drive wire (158).

9. The forceps device of claim 8, the drive wire (158) comprising Nitinol.

10. The forceps device of claim 1 comprising nickel.

11. The forceps device of claim 1, wherein the device is grown via Physical Vapor Deposition.

## Patentansprüche

1. Zangenvorrichtung (100), umfassend:
eine Nadel (106); und
eine Zangenbaugruppe (102), aufweisend
eine erste Backe (110a, 300), umfassend zwei beabstandete Wände (115a, 115b) und eine zylindrische Säule (117), die sich zwischen den Innenseiten der Wände (115a) erstreckt,
eine zweite Backe (110b, 400), die der ersten Backe gegenüberliegt und durch einen Gabelkopf (150) mit dieser drehbar verbunden ist,
ein erstes Verbindungsstück (120), das drehbar mit der ersten Backe verbunden ist,
ein zweites Verbindungsstück (130), das drehbar mit der zweiten Backe verbunden ist, und
einen Kastenschieber (140), der sowohl mit dem ersten als auch dem zweiten Verbindungsstück drehbar verbunden ist und ein Paar Verbindungsstücke (142a, 142b) umfasst, die jeweils ein Loch aufweisen;
wobei das erste Verbindungsstück (120) ein erstes Verbindungsloch (122) und ein zweites Verbindungsloch (124) umfasst, wobei das zweite Verbindungsstück (130) Verbindungsstifte (132, 134) umfasst; wobei die Verbindungsstifte (132, 134) für die Verbindung mit dem zweiten Verbindungsloch (124) und den Verbindungsstücken (142a, 142b) konfiguriert sind,
wobei das erste Verbindungsloch (122) drehbar mit der zylindrischen Säule (117) der ersten Backe verbunden ist,
wobei der Kastenschieber (140) mindestens ein Führungselement (144) umfasst, das so dimensioniert ist, dass es in einen Führungskanal (152) eines Gabelkopfes (150), in dem der Kastenschieber angeordnet ist, passt und horizontal darin gleitet.
wobei das Bewegen des Kastenschiebers (140) in distaler Richtung bewirkt, dass sich die erste und die zweite Backe in eine offene Position drehen, das Bewegen des Kastenschiebers (140) in proximaler Richtung bewirkt, dass sich die erste und die zweite Backe in eine geschlossene Position drehen, und die Backen dazu konfiguriert sind, aus dem Inneren der Nadel (106) heraus eingesetzt zu werden; und
wobei die Zangenvorrichtung (100) dazu konfiguriert ist, aus einem Lumen, einer Kanüle, einem Katheter, einem Endoskop oder einem Laparoskop heraus eingesetzt zu werden.

2. Zangenvorrichtung nach Anspruch 1, wobei die Backen glatte Schalen umfassen.

3. Zangenvorrichtung nach Anspruch 1, wobei jede Backe mindestens einen Zahn (302, 402) umfasst.

4. Zangenvorrichtung nach Anspruch 3, wobei der mindestens eine Zahn jeder Backe eine Vielzahl von Zähnen beinhaltet, deren Länge von einem proximalen Ende der Backe (300, 400) zu einem distalen Ende der Backe (300, 400) zunimmt.

5. Zangenvorrichtung nach Anspruch 4,
die erste Backe (300) aufweisend
einen ersten Zahn (302);
einen zweiten Zahn (304) distal zum ersten Zahn und länger als dieser,
einen dritten Zahn (306) distal zum zweiten Zahn und länger als dieser,
einen vierten Zahn (308) distal zum dritten Zahn und von gleicher Länge wie dieser,
einen fünften Zahn (310) distal zum vierten Zahn und länger als dieser,
einen sechsten Zahn (312) distal zum fünften Zahn und länger als dieser,
einen siebten Zahn (314) distal zum sechsten Zahn und länger als dieser, und
einen achten Zahn (316) distal zum siebten Zahn und länger als dieser; und
die zweite Backe (400) aufweisend
einen ersten Zahn (402), der länger als der erste Zahn der ersten Backe ist;
einen zweiten Zahn (404) distal zum ersten Zahn und von gleicher Länge wie dieser,
einen dritten Zahn (406), distal zum zweiten Zahn und länger als dieser,
einen vierten Zahn (408) distal zum dritten Zahn und länger als dieser ist,
einen fünften Zahn (410) distal zum vierten Zahn und länger als dieser,
einen sechsten Zahn (412) distal zum fünften Zahn und länger als dieser,
einen siebten Zahn (414) distal zum sechsten Zahn und länger als dieser, und
einen achten Zahn (416) distal zum siebten Zahn und von gleicher Länge wie dieser.

6. Zangenvorrichtung nach Anspruch 1, wobei mindestens eine der ersten und zweiten Backe eine Fensterung (112) aufweist.

7. Zangenvorrichtung nach Anspruch 6, wobei ein distales Ende der Fensterung (112) in Bezug auf eine Außenfläche der Backe, die die Fensterung (112) aufweist, abgewinkelt ist.

8. Zangenvorrichtung nach Anspruch 1, wobei der Kastenschieber (140) mit einem Antriebsdraht (158) verbunden ist.

9. Zangenvorrichtung nach Anspruch 8, wobei der Antriebsdraht (158) Nitinol umfasst.

10. Zangenvorrichtung nach Anspruch 1, umfassend Nickel.

11. Zangenvorrichtung nach Anspruch 1, wobei die Vorrichtung durch physikalische Gasphasenabscheidung gebildet wird.

## Revendications

1. Dispositif de pinces (100), comprenant :
une aiguille (106) ; et
un ensemble de pinces (102) ayant
une première mâchoire (110a, 300) comprenant deux parois espacées (115a, 115b) et un pilier cylindrique (117) s'étendant entre les côtés intérieurs desdites parois (115a),
une seconde mâchoire (110b, 400) opposée et reliée en rotation à la première mâchoire par une chape (150),
une première pièce de liaison (120) reliée en rotation à la première mâchoire,
une seconde pièce de liaison (130) reliée en rotation à la seconde mâchoire, et
un coulisseau de boîtier (140) relié en rotation à la fois aux première et seconde pièces de liaison et comprenant une paire d'éléments de liaison (142a, 142b), chacun ayant un trou ;
dans lequel la première pièce de liaison (120) comprend un premier trou de liaison (122) et un second trou de liaison (124), dans lequel la seconde pièce de liaison (130) comprend des broches de liaison (132, 134) ; dans lequel lesdites broches de liaison (132, 134) sont configurées pour la connexion au second trou de liaison (124) et aux éléments de liaison (142a, 142b),
dans lequel le premier trou de liaison (122) est relié en rotation au pilier cylindrique (117) de la première mâchoire,
dans lequel le coulisseau de boîtier (140) comprend au moins un élément de guidage (144) dimensionné pour s'ajuster et coulisser horizontalement dans un canal de guidage (152) d'une chape (150) dans laquelle le coulisseau de boîtier est placé,
dans lequel le déplacement du coulisseau de boîtier (140) fait tourner de manière distale les première et seconde mâchoires vers une position ouverte, le déplacement du coulisseau de boîtier (140) fait tourner de manière proximale les première et seconde mâchoires vers une position fermée, et les mâchoires sont configurées pour être déployées depuis l'intérieur de l'aiguille (106) ; et
dans lequel le dispositif de pinces (100) est configuré pour être déployé depuis l'intérieur d'une lumière, d'une canule, d'un cathéter, d'un endoscope ou d'un laparoscope.

2. Dispositif de pinces selon la revendication 1, les mâchoires comportant des coupelles lisses.

3. Dispositif de pinces selon la revendication 1, chaque mâchoire comprenant au moins une dent (302, 402).

4. Dispositif de pinces selon la revendication 3, dans lequel l'au moins une dent de chaque mâchoire comprend une pluralité de dents dont la longueur augmente d'une extrémité proximale de la mâchoire (300, 400) à une extrémité distale de la mâchoire (300, 400).

5. Dispositif de pinces selon la revendication 4,
la première mâchoire (300) ayant
une première dent (302) ;
une deuxième dent (304) distale et plus longue que la première dent,
une troisième dent (306) distale et plus longue que la deuxième dent,
une quatrième dent (308) distale et de même longueur que la troisième dent,
une cinquième dent (310) distale et plus longue que la quatrième dent,
une sixième dent (312) distale et plus longue que la cinquième dent,
une septième dent (314) distale et plus longue que la sixième dent, et
une huitième dent (316) distale et plus longue que la septième dent ; et
la seconde mâchoire (400) ayant
une première dent (402) plus longue que la première dent de la première mâchoire ;
une deuxième dent (404) distale et de même longueur que la première dent,
une troisième dent (406) distale et plus longue que la deuxième dent,
une quatrième dent (408) distale et plus longue que la troisième dent,
une cinquième dent (410) distale et plus longue que la quatrième dent,
une sixième dent (412) distale et plus longue que la cinquième dent,
une septième dent (414) distale et plus longue que la sixième dent, et
une huitième dent (416) distale et de même longueur que la septième dent.

6. Dispositif de pinces selon la revendication 1, au moins l'une des première et seconde mâchoires comportant une fenêtre (112).

7. Dispositif de pinces selon la revendication 6, dans lequel une extrémité distale de la fenêtre (112) est inclinée par rapport à une surface externe de la mâchoire ayant la fenêtre (112) .

8. Dispositif de pinces selon la revendication 1, le coulisseau de boîtier (140) étant relié à un fil d'entraînement (158).

9. Dispositif de pinces selon la revendication 8, le fil d'entraînement (158) comprenant du Nitinol.

10. Dispositif de pinces selon la revendication 1, comprenant du nickel.

11. Dispositif de pinces selon la revendication 1, dans lequel le dispositif est développé par dépôt physique en phase vapeur.
